# EUROPEAN PATENT APPLICATION

(11) **EP 4 840 843 A1**
(43) Date of publication of application: **18.11.2026**
(21) Application number: 25738405.7
(22) Date of filing: 07.01.2025
(51) Int. Cl.: C07C 217/18, C07C 213/02, A61P 29/00

(54) **PTEROSTILBENE COMPOUND, PHARMACEUTICAL COMPOSITION AND USE THEREOF IN PREPARATION OF ANTI-INFLAMMATORY DRUGS**

(30) Priority: 12.01.2024 CN 202410049666
(71) Applicant: Accendatech (Tianjin) Biopharmaceutical Co., Ltd., Tianjin 300192 (CN)
(72) Inventor: FENG, Peng, Tianjin 300192 (CN); BAO, Shiqi, Tianjin 300192 (CN); ZHANG, Xuemei, Tianjin 300192 (CN); ZHU, Lin, Tianjin 300192 (CN)
(74) Representative: Gille Hrabal Partnerschaftsgesellschaft mbB Patentanwälte
(86) International application number: PCT/CN2025/071047
(87) International publication number: WO 2025/148875

(57) **Abstract**

The compound of formula (I), or a pharmaceutically acceptable salt thereof, provided by the present invention has high stability and high bioavailability, can effectively improve symptoms of DSS-induced colitis in mice, such as colon shortening, diarrhea, hematochezia and weight loss, has a significant inhibitory effect on intestinal inflammation, and can be used for preparation anti-inflammatory drugs.

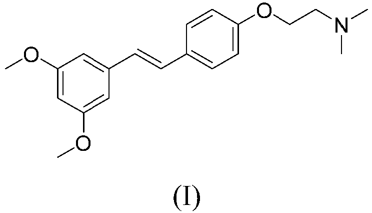

## Description

This application claims the benefit of priority from an earlier application filed by the applicant with the China National Intellectual Property Administration on January 12, 2024, with the application number 202410049666.X and entitled " PTEROSTILBENE COMPOUNDS, PHARMACEUTICAL COMPOSITIONS THEREOF, AND USE THEREOF IN PREPARATION OF ANTI-INFLAMMATORY DRUGS"; the entire content of the earlier application is incorporated herein by reference.

### TECHNICAL FIELD

The present disclosure belongs to the field of medicine, and specifically relates to a pterostilbene compound, a pharmaceutical composition thereof, and use thereof in the preparation of anti-inflammatory drugs.

### BACKGROUND

Inflammatory bowel disease (IBD) is a common chronic inflammatory disease of the gastrointestinal tract. The etiology of IBD is very complex, and its pathogenesis remains unclear. Current research indicates that IBD may be influenced by a combination of multiple factors, such as host genetic susceptibility, gut microbiota, environmental factors, and immune abnormalities. To date, the main clinical drugs for IBD treatment are classic anti-inflammatory drugs, immunosuppressive drugs, and some antibody drugs. There is still no effective drug that can completely cure IBD. The goal of clinical treatment is to alleviate symptoms, maintain an asymptomatic state, and prevent recurrence. Therefore, developing new therapeutic drugs with minimal side effects and definite efficacy has profound clinical application prospects and significant social importance.

### SUMMARY

The present disclosure provides a compound represented by formula (I) or a pharmaceutically acceptable salt thereof:

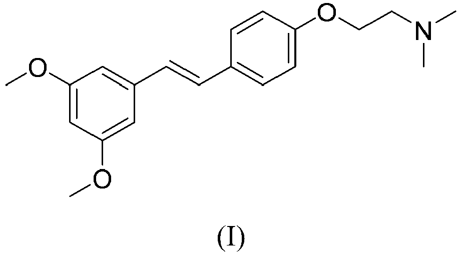

According to an embodiment of the present disclosure, the pharmaceutically acceptable salt can be an acid addition salt, for example, an acid addition salt formed from the compound represented by formula (I) and an inorganic acid such as hydrochloric acid, hydrofluoric acid, hydrobromic acid, hydriodic acid, sulfuric acid, pyrosulfuric acid, phosphoric acid, or nitric acid, or a bisulfate salt; or an acid addition salt formed with an organic acid such as formic acid, acetic acid, acetoacetic acid, pyruvic acid, trifluoroacetic acid, propionic acid, butyric acid, hexanoic acid, heptanoic acid, undecanoic acid, lauric acid, benzoic acid, salicylic acid, 2-(4-hydroxybenzoyl)benzoic acid, camphoric acid, cinnamic acid, cyclopentanepropionic acid, digluconic acid, 3-hydroxy-2-naphthoic acid, nicotinic acid, pamoic acid, pectinic acid, persulfuric acid, 3-phenylpropionic acid, picric acid, pivalic acid, 2-hydroxyethanesulfonic acid, itaconic acid, sulfamic acid, trifluoromethanesulfonic acid, dodecyl sulfate, ethanesulfonic acid, benzenesulfonic acid, p-toluenesulfonic acid, methanesulfonic acid, 2-naphthalenesulfonic acid, naphthalenedisulfonic acid, camphorsulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptonic acid, glycerophosphoric acid, aspartic acid, sulfosalicylic acid, hemisulfuric acid, or thiocyanic acid; preferably, it is fumaric acid.

According to an embodiment of the present disclosure, the structure of the pharmaceutically acceptable salt is as follows:

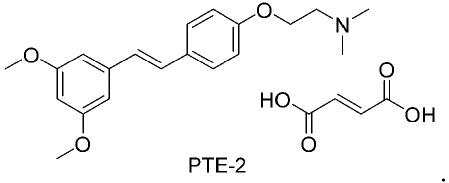

The present disclosure also provides a method for preparing the compound of formula (I), comprising the step of: reacting compound 1 with compound 2 to obtain the compound of formula (I); 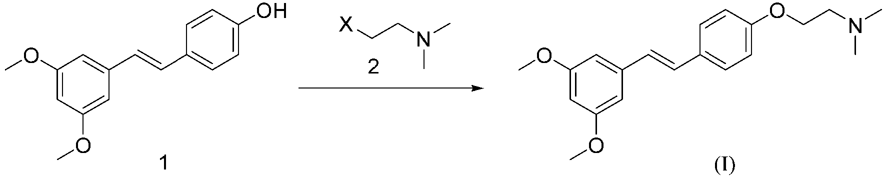 wherein X is halogen, for example Cl, Br, and I.

The present disclosure also provides a method for preparing a pharmaceutically acceptable salt of the compound of formula (I), comprising the step of: mixing the compound of formula (I) with an acid in a solvent to obtain the pharmaceutically acceptable salt of the compound of formula (I); the acid has the definition as described above.

According to an embodiment of the present disclosure, the preparation method comprises dissolving the compound of formula (I) in a solvent, then adding an acid and continuing the reaction to obtain the pharmaceutically acceptable salt of the compound of formula (I).

According to an embodiment of the present disclosure, the preparation method comprises dissolving the compound of formula (I) in ethyl acetate, adding fumaric acid, and reacting to obtain the fumarate salt of the compound of formula (I).

A pharmaceutical composition, comprising the compound of formula (I) or a pharmaceutically acceptable salt thereof.

The present disclosure also provides use of the compound of formula (I) or a pharmaceutically acceptable salt thereof or the pharmaceutical composition in the preparation of an anti-inflammatory drug, for example, in the preparation of a drug for treating inflammatory bowel disease.

A method for treating an inflammatory disease, comprising administering to a patient a therapeutically effective amount of the compound of formula (I) or a pharmaceutically acceptable salt thereof or the pharmaceutical composition.

According to an embodiment of the present disclosure, the inflammatory disease is inflammatory bowel disease.

The present disclosure also provides the compound of formula (I) or a pharmaceutically acceptable salt thereof or the pharmaceutical composition for treating an inflammatory disease.

### BRIEF DESCRIPTION OF THE DRAWINGS

FIG. 1 Effect of PTE-2 on the DAI score in a DSS-induced mouse enteritis model (DAI score = (weight loss score + stool consistency score + fecal bleeding score)/3; "^{***}" indicates *P* < 0.001 compared with the blank control group; "^{##}"indicates *P* < 0.01 compared with the model control group).
FIG. 2 Effect of PTE-2 on the colorectal length in a DSS-induced mouse enteritis model ("^{***}" indicates *P* < 0.001 compared with the blank control group; "##"indicates P < 0.01 compared with the model control group).
FIG. 3 Effect of PTE-2 on the HE staining score in a DSS-induced mouse enteritis model ("^{***}" indicates *P* < 0.001 compared with the blank control group; "#" indicates P < 0.05 compared with the model control group).
FIG. 4 Effect of PTE-2 on the body weight in a DSS-induced mouse enteritis model ("^{***}" indicates *P* < 0.001 compared with the blank control group; "#" indicates P < 0.05 compared with the model control group).

### Advantageous Effects

The compound of formula (I) or a pharmaceutically acceptable salt thereof provided by the present disclosure has high stability and high bioavailability, can effectively improve symptoms such as colon shortening, diarrhea, bloody stools, and weight loss caused by DSS-induced mouse colitis, has a significant inhibitory effect on intestinal inflammation, and can be used in the preparation of anti-inflammatory drugs (e.g., drugs for treating inflammatory bowel disease).

### DETAILED DESCRIPTION

The technical solutions of the present disclosure are further described in detail below with reference to specific examples. It will be appreciated that the following examples are merely exemplary illustrations and explanations of the present disclosure and should not be construed as limiting the claimed scope of the present disclosure. All techniques implemented on the basis of the content described above of the present disclosure fall within the claimed scope of the present disclosure.

Unless otherwise stated, the starting materials and reagents used in the following examples are all commercially available products or can be prepared by using known methods.

### Example 1

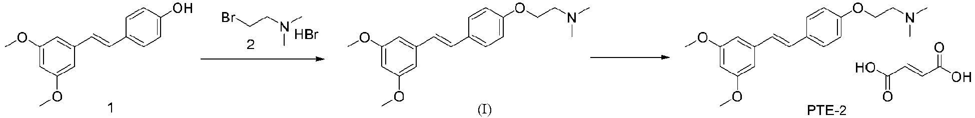

Pterostilbene (Compound 1, 23.43 g, 1.0 eq) and 2-(dimethylamino)ethyl bromide hydrobromide (31.17 g, 1.5 eq) were weighed and added to a round-bottom flask, and DMF (560 mL) was added for dissolution. Under nitrogen protection, the system was cooled to 0 °C, sodium hydride (12.06 g, 3.0 eq) was weighed and added to the reaction mixture, after which the system was gradually warmed to room temperature and stirring was continued overnight. After TLC detection indicated that most of the starting material had disappeared, water was added to quench the reaction, the pH of the system was adjusted to 2-3 with 1 N HCl solution, and ethyl acetate was added for extraction and phase separation; the organic phase was discarded. The pH of the aqueous phase was adjusted to 8-9 with a saturated potassium carbonate solution, extraction was performed with ethyl acetate, and the organic phase was washed with saturated brine and dried over anhydrous sodium sulfate. The mixture was filtered under suction and concentrated under reduced pressure to afford 17.52 g of the compound of formula (I) as a light brown oil, yield: 58.52 %, which was used directly in the next step. ¹H NMR (600 MHz, DMSO) δ 7.51 (d, *J =* 9.0 Hz, 2H), 7.20 (d, *J =* 16.2 Hz, 1H), 7.02 (d, *J =* 16.2 Hz, 1H), 6.95 (d, *J =* 9.0 Hz, 2H), 6.74 (d, *J =* 2.4 Hz, 2H), 6.39 (t, *J =* 2.4 Hz, 1H), 4.06 (t, *J =* 6.0 Hz, 2H), 3.77 (s, 6H), 2.62 (t, *J =* 6.0 Hz, 2H), 2.21 (s, 6H).

The compound shown in formula (I) (17.52 g, 1.0 eq) was dissolved in ethyl acetate (100 mL), fumaric acid (3.22 g, 0.9 eq) was added, the system was heated to 50 °C, and stirring was continued for 5 hours. The mixture was then cooled to room temperature and filtered to obtain 16.27 g of a white powder (PTE-2), yield: 76.20%. ¹H NMR (600 MHz, DMSO) δ 7.53 (d, *J =* 9.0 Hz, 2H), 7.21 (d, *J =* 16.2 Hz, 1H), 7.03 (d, *J =* 16.2 Hz, 1H), 6.96 (d, *J =* 9.0 Hz, 2H), 6.74 (d, *J =* 2.4 Hz, 2H), 6.58 (s, 2H), 6.39 (t, *J =* 2.4 Hz, 1H), 4.16 (t, *J =* 5.4 Hz, 2H), 3.77 (s, 6H), 2.91 (t, *J =* 5.4 Hz, 2H), 2.42 (s, 6H).

### Example 2 Stability Test

The test sample was placed in a weighing bottle, dispersed, with a thickness not exceeding 3 mm. Tests were conducted under high temperature, high humidity, and illumination conditions, respectively.
(1) High temperature test: The test sample was placed open in a suitable constant temperature device, the temperature was set at 60 °C, the sampling time points were set at 0 days, 5 days, and 10 days to investigate the changes in related substances.
(2) High humidity test: The test sample was placed open in a constant humidity airtight container, at 25 °C, under conditions of relative humidity 90% ± 5% (KNO₃ saturated solution (relative humidity 92.5%, 25 °C)) for 10 days, samples were taken on day 5 and day 10 to investigate the changes in related substances.
(3) Strong light irradiation test: The test sample was placed open in a light chamber, simultaneously exposed to cool white fluorescent lamps and near-ultraviolet lamps, under an illumination intensity of 4500 lx ± 500 lx, and the total illumination of the light source should be not less than 1.2×10⁶ lux·hr, the near-ultraviolet lamp energy should be not less than 200 W·hr/m², samples were taken on day 5 and day 10 to investigate the changes in related substances.

Related substance detection method (HPLC):
Mobile phase A: H₂O : ACN : H₃PO₄ = 90 : 10 : 0.1;
Mobile phase B: H₂O : ACN : H₃PO₄ = 10 : 90 : 0.1;
Column: Octadecylsilane-bonded silica gel was used as the filler, 4.6*250 mm, 5 µm;
Gradient:

| Time | B% |
|---|---|
| 0 | 10 |
| 20 | 90 |
| 25 | 90 |
| 26 | 10 |
| 32 | 10 |

Test sample solution: An appropriate amount of the product was taken, dissolved and diluted with 10% methanol to prepare a solution containing about 5 mg per 1 mL, and 10 µL was injected.

The test results are as follows:

| RRT | 0 | High Temperature, 5 Days | High Temperature, 10 Days | High Humidity, 5 Days | High Humidity, 10 Days | Light Exposure, 5 Days | Light Exposure, 10 Days |
|---|---|---|---|---|---|---|---|
| 0.54 | N.D. | N.D. | N.D. | N.D. | N.D. | 0.03 | 0.09 |
| 0.75 | N.D. | N.D. | N.D. | N.D. | N.D. | N.D. | 0.031 |
| 0.85 | N.D. | N.D. | N.D. | N.D. | N.D. | N.D. | 0.026 |
| 0.87 | N.D. | N.D. | N.D. | N.D. | N.D. | N.D. | 0.100 |
| 0.91 | N.D. | N.D. | N.D. | N.D. | N.D. | N.D. | 0.041 |
| 0.99 | 0.023 | 0.032 | 0.035 | 0.026 | 0.027 | 0.052 | 0.067 |
| 1 | 99.466 | 99.393 | 99.394 | 99.415 | 99.416 | 99.351 | 99.017 |
| 1.05 | 0.046 | 0.070 | 0.080 | 0.066 | 0.061 | 0.056 | 0.068 |
| 1.08 | 0.029 | 0.035 | 0.057 | 0.057 | 0.049 | 0.025 | 0.082 |
| 1.09 | 0.0436 | 0.431 | 0.434 | 0.436 | 0.447 | 0.426 | 0.437 |
| 1.29 | N.D. | N.D. | N.D. | N.D. | N.D. | 0.024 | 0.040 |

The results showed that the compound of the present disclosure was relatively stable under high temperature and high humidity conditions but was sensitive to light.

### Example 3

### 1. Experimental Method

(1) The mice were randomly grouped according to body weight, with 10 mice per group, totaling 4 groups. One group of mice was randomly selected as the blank control group and given normal drinking water. For the remaining mice, a DSS-induced mouse enteritis model was established: DSS powder was dissolved in ultrapure water at a concentration of 3% (3 g of DSS was added to 100 mL of ultrapure water), and the mice were allowed to drink freely, with the water changed every other day. The day of model establishment was designated as day 1 of the experiment. The remaining 8 groups of mice were randomly grouped, and feces were collected every two days and stored frozen at -80°C. The body weight of the mice was measured daily, and the drug was administered by gavage according to body weight, while the condition and feces of the mice were observed. The experiment was terminated after 9 consecutive days of drug administration. The mice were dissected to observe intestinal conditions and samples were collected.
(2) The colorectum was taken, its length was measured and photographed. A sample from the distal 2 cm of the colon was fixed in paraformaldehyde and blindly sent to a testing company for HE staining and scoring.

### 2. Experimental Groups:

(1) Blank control group: normal drinking water;
(2) Model control group: DSS drinking water for 9 days;
(3) Pterostilbene-100 mg/kg group, DSS drinking water for 9 days;
(4) PTE-2-100 mg/kg group, DSS drinking water for 9 days;
   10 mice per group, 40 mice in total.

### 3. Drug Preparation and Administration Method

Pterostilbene, dissolved in corn oil, sonicated for approximately 15 minutes, administered by gavage, once daily;
Compound PTE-2, dissolved in physiological saline, sonicated for approximately 15 minutes, administered by gavage, once daily;
The blank control group and the model control group were administered an equal volume of physiological saline.

### 4. Experimental Results and Analysis

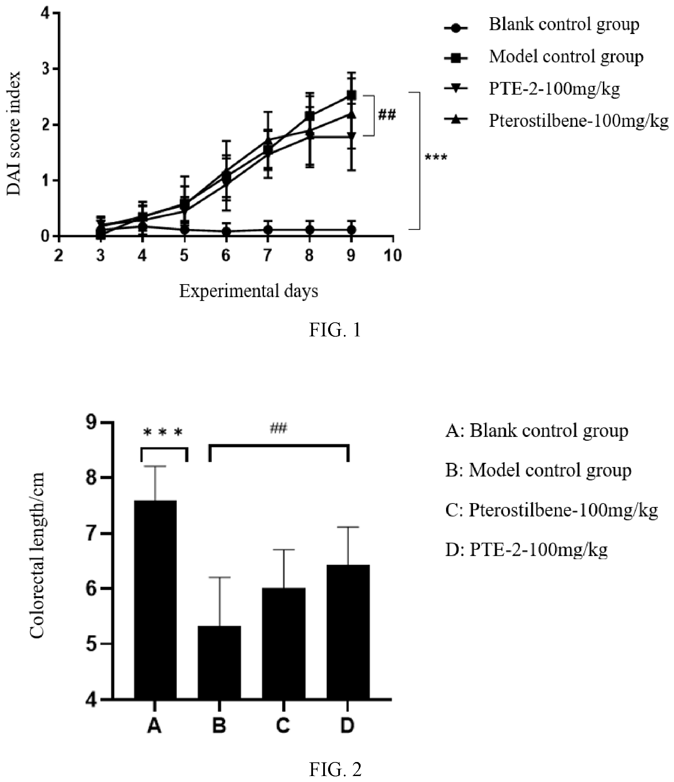

The experimental results are shown in Figures 1 to 4:
As shown in Figure 1, starting from the fourth day of administration, the animals generally began to exhibit varying degrees of weight loss and diarrhea. The DAI score was calculated based on the percentage of weight loss, fecal condition, and intestinal bleeding. By the end of the experiment, the DAI score of the model control group was significantly higher than that of the blank control group (*P*<0.001); the DAI score of the model control group was (2.5±0.4), the DAI score of the pterostilbene-100 mg/kg group was (2.2±0.6), and the DAI score of the PTE-2-100 mg/kg group was (1.8±0.6) (compared with the model control group, *P*<0.01). This indicates that PTE-2 can significantly ameliorate symptoms such as diarrhea and bloody stools in mice with colitis induced by DSS.

As shown in Figure 2, DSS-induced acute colitis leads to shortening of the colon in the model animals. Compared with the blank control group, the colorectum of the model control group was significantly shortened (*P*<0.001). The colorectum length of the model control group was (5.3±0.9 cm), that of the pterostilbene-100 mg/kg group was (6.0±0.7 cm), and that of the PTE-2-100 mg/kg group was (6.4±0.7 cm) (compared with the model control group, *P*<0.01). This indicates that PTE-2 can significantly inhibit colon shortening in mice with DSS-induced acute colitis.

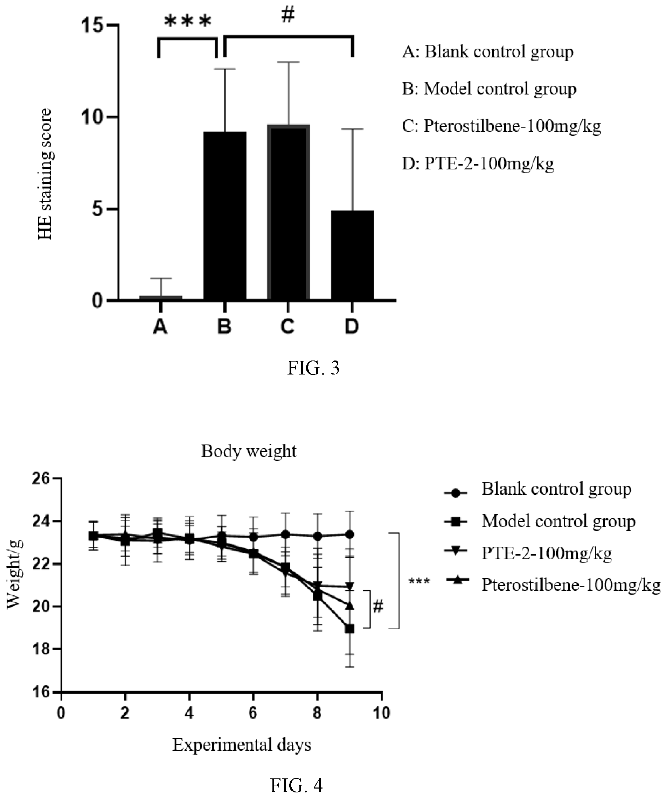

As shown in Figure 3, compared with the blank control group, the HE staining score of the model control group was significantly increased (*P*<0.001). The HE staining score of the model control group was (9.2±3.4), that of the pterostilbene-100 mg/kg group was (9.6±3.4), and that of the PTE-2-100 mg/kg group was (4.9±4.5) (compared with the model control group, *P*<0.05). This indicates that PTE-2 can effectively improve intestinal inflammation and pathological changes in mice with DSS-induced acute colitis.

As shown in Figure 4, compared with the blank control group, the body weight of the model control group decreased significantly (*P*<0.001). The body weight of the model control group was (19.0±1.8 g), and that of the PTE-2-100 mg/kg group was (20.9±1.8 g) (compared with the model control group, *P*<0.05). The results indicate that PTE-2 can effectively alleviate the body weight loss in mice with DSS-induced acute colitis.

The experimental results are summarized in Table 1 below:

**Table 1 Preventive effect of PTE-2 on the DSS-induced mouse enteritis model (x±s)**

| Group | Adminis tration route and frequenc y | Number (n) | | Body weight (g) | | DAI score | | Colorectal length (cm) | HE staining score |
|---|---|---|---|---|---|---|---|---|---|
| | | Start | End | Day 1 of administra tion | End | First day of symptom onset | End | | |
| Blank control group | p.o.×9 | 10 | 10 | 23.3±0.7 | 23.4±1.1 | 0.2±0.2 | 0.1±0.2 | 7.6±0.6 | 0.3±0.9 |
| Model control group | p.o.×9 | 10 | 10 | 23.3±0.7 | 19.0±1.8*** | 0.4±0.2 | 2.5±0.4*** | 5.3±0.9*** | 9.2±3.4*** |
| Pterostilbene-100 mg/kg group | p.o.×9 | 10 | 10 | 23.4±0.6 | 20.1±2.3 | 0.4±0.2 | 2.2±0.6 | 6.0±0.7 | 9.6±3.4 |
| PTE-2-100 mg/kg group | p.o.×9 | 10 | 10 | 23.3±0.7 | 20.9±1.8 ^{#} | 0.3±0.2 | 1.8±0.6 ^{##} | 6.4±0.7 ^{##} | 4.9±4.5^{#} |

| | | | | | | | | | |
|---|---|---|---|---|---|---|---|---|---|
| Note: The score is (weight loss score + fecal score + bloody stool score)/3; "###" indicates P < 0.001 compared with the blank control group; "*" indicates P < 0.05 compared with the model control group; "***" indicates P < 0.001 compared with the model control group. | | | | | | | | | |

### Result Analysis:

Compared with the blank control group, the colorectum of the model control group was significantly shortened (P<0.001). The colorectum length of the model control group was (5.3±0.9 cm), and that of the PTE-2-100 mg/kg group was (6.4±0.7 cm) (*P* < 0.01), while the pterostilbene-100 mg/kg group showed no statistical difference.

The DAI score of the model control group was (2.5±0.4), and that of the PTE-2-100 mg/kg group was (1.8±0.6), which was significantly reduced compared with the model control group (*P* < 0.01).

HE staining results showed that the score of the model control group was (9.2±3.4), which was significantly different from that of the blank control group (0.3±0.9) (*P* < 0.001); compared with the model control group, the score of the PTE-2-100 mg/kg group (4.9±4.5) showed a statistical difference (*P* < 0.05).

Through the above analysis, it was found that PTE-2 can effectively ameliorate symptoms such as colon shortening, diarrhea, bloody stool, and weight loss induced by DSS in mouse colitis, and has a significant inhibitory effect on intestinal inflammation and pathological changes.

Example 4 Distribution Experiment of PTE-2 and Pterostilbene in Brain and Other Tissues
(1) 102 healthy Balb/c mice weighing about 20 g were taken and divided into 5 groups, with 12 mice in the blank group.
   A. PTE-2 oral gavage group (dosage: 100 mg/kg): 21 mice;
   B. PTE-2 tail vein injection group (dosage: 20 mg/kg): 24 mice.
   C. Pterostilbene oral gavage group (dosage: 100 mg/kg): 21 mice;
   D. Pterostilbene tail vein injection group (dosage: 20 mg/kg): 24 mice.
   E. Blank plasma was taken as a blank matrix, 12 mice.
(2) For the oral gavage group, blood samples were collected at 5 min, 15 min, 30 min, 1 h, 3 h, 8 h, and 24 h after administration. For the intravenous injection group, blood samples were collected at 2 min, 5 min, 15 min, 30 min, 1 h, 3 h, 8 h, and 24 h after administration. Blood was collected from mice via eyeball removal and placed in anticoagulant tubes containing sodium heparin. The samples were immediately centrifuged at 5000 rpm at low temperature (4°C) for 10 min to obtain plasma samples. The samples were stored in a -80°C freezer for analysis. Three mice were used per time point.
(3) The mouse was fixed on a foam board. The skin on the chest was lifted with forceps, and the thoracic skin and ribs were cut open with scissors to expose the heart and liver. The auricle was then cut open. A syringe needle was inserted into the left ventricle of the mouse, and normal saline was perfused for approximately 1 min (10-20 mL) until the mouse's limbs, liver, and tongue turned pale. Brain collection: The skin on the head was cut open to expose the white skull. The cartilage was cut, the skull cap was carefully pried open to expose the white brain, and the entire brain was dissected out. Simultaneously, tissues such as the pancreas, liver, and lung were collected (only brain tissue was collected for the pterostilbene administration group). The samples were stored in a -80°C freezer, awaiting analysis.
(4) The contents of PTE-2 and pterostilbene in the tissues were detected by LC/MS.

### Main Instruments

ExionLC high performance liquid chromatography system, SCIEX;
Triple Quad^{™} 4500 tandem triple quadrupole mass spectrometer, SCIEX;
MS105DU microbalance (1/100,000), Mettler-Toledo;
S1-0256 circular oscillating mixer, IKA;
JXFSTPRP-CL cryogenic tissue grinder, Shanghai Jingxin;
Eppendorf Centrifuge 5425R compact benchtop high-speed refrigerated centrifuge, Eppendorf;

### Detection Conditions

| **Liquid chromatography conditions:** | | | | | | |
|---|---|---|---|---|---|---|
| Autosampler: | ExionLC-AD | | | | | |
| Liquid Chromatograph: | ExionLC-AD | | | | | |
| Column Oven: | ExionLC-AC | | | | | |
| System Control Module: | ExionLC | | | | | |
| Chromatographic column: | CORTECS^{®} C18, 2.7 µm (2.1×50 mm) | | | | | |
| Column Temperature: | 40°C | | | | | |
| Injection volume: | PTE-2: 2 µL for brain tissue; 1 µL for other tissues (liver, lung, pancreas); Pterostilbene: 8 µL | | | | | |
| Mobile phase: | A: H₂O (containing 0.1% formic acid) | | | | | |
| | B: Acetonitrile (containing 0.1% formic acid) | | | | | |

| PTE-2 Gradient: | Time (min) | A (%) | | B (%) | Flow Rate (mL/min) | |
|---|---|---|---|---|---|---|
| | 0 | 95 | | 5 | 0.6 | |
| | 1.0 | 0 | | 100 | 0.6 | |
| | 1.5 | 0 | | 100 | 0.6 | |
| | 1.6 | 95 | | 5 | 0.6 | |
| | 2.0 | 95 | | 5 | 0.6 | |
| | | | | | | |

| Pterostilbene gradient: | Time (min) | A (%) | | B (%) | Flow Rate (mL/min) | |
|---|---|---|---|---|---|---|
| | 0 | 90 | | 10 | 0.4 | |
| | 0.3 | 90 | | 10 | 0.4 | |
| | 1.5 | 0 | | 100 | 0.4 | |
| | 2.0 | 0 | | 100 | 0.4 | |
| | 2.1 | 90 | | 10 | 0.4 | |
| | 3.0 | 90 | | 10 | 0.4 | |

| **Mass spectrometry conditions:** | | | | | | |
|---|---|---|---|---|---|---|
| Instrument: | Triple Quad^{™} 4500 (Sciex; Framingham, MA, USA) | | | | | |
| Ion source: | Electrospray ionization source | | | | | |
| Ionization mode: | Electrospray | | | | | |
| Scanning mode: | MRM | | | | | |
| Ion mode: | PTE-2: positive ion mode; Pterostilbene: negative ion mode | | | | | |

| MS/MS information | | | | | | |
|---|---|---|---|---|---|---|
| Analyte | Q1 (m/z) | Q3 (m/z) | DP (v) | EP (v) | CE (v) | CXP (v) |
| PTE-2 | 328.1 | 72.2 | 95 | 10 | 40 | 15 |
| Pterostilbene | 255.0 | 196.8 | -94 | -10 | -41 | -11 |
| Tolbutamide (IS) | 271.3 | 155.0 | 88 | 10 | 17 | 15 |
| Tolbutamide (IS) | 269.1 | 169.7 | -68 | -10 | -23 | -11 |

| | | | | | | |
|---|---|---|---|---|---|---|
| Note: Tolbutamide was used as the internal standard for calculating the peak area ratio. | | | | | | |

### Test Results

A comparison of the exposure levels in plasma and tissues following single-dose oral gavage and intravenous administration of PTE-2 and pterostilbene is shown in the table below.

| Compound | Plasma or Tissue | AUC₀₋ₜ (hr*ng/mL, hr*ng/g) | | Fold ( AUCₜᵢₛₛᵤₑ/AUCₚₗₐₛₘₐ ) | |
|---|---|---|---|---|---|
| | | iv (20mg/kg) | po (100mg/kg) | iv (20mg/kg) | po (100mg/kg) |
| PTE-2 | Plasma | 1134 | 1585 | - | - |
| | Brain | 55058 | 42230 | 49 | 27 |
| | Liver | 117789 | 802349 | 104 | 506 |
| | Lung | 29414 | 82118 | 26 | 52 |
| | Pancreas | 46349 | 209482 | 41 | 132 |
| Pterostilbe ne | Plasma | 4265 | 2357 | - | - |
| | Brain | 8607 | 5830 | 2.02 | 2.47 |

Example 5 Pharmacokinetic Study of PTE-2 and Pterostilbene
(1) 102 healthy Balb/c mice weighing about 20 g were taken and divided into 5 groups, with 12 mice in the blank control group.
   A. PTE-2 oral gavage group (dosage: 100 mg/kg): 21 mice;
   B. PTE-2 tail vein injection group (dosage: 20 mg/kg): 24 mice.
   C. Pterostilbene oral gavage group (dosage: 100 mg/kg): 21 mice;
   D. Pterostilbene tail vein injection group (dosage: 20 mg/kg): 24 mice.
   E. Blank plasma was taken as a blank matrix, 12 mice.
(2) For the oral gavage group, blood samples were collected at 5 min, 15 min, 30 min, 1 h, 3 h, 8 h, and 24 h after administration. For the intravenous injection group, blood samples were collected at 2 min, 5 min, 15 min, 30 min, 1 h, 3 h, 8 h, and 24 h after administration. Blood was collected from mice via eyeball removal and placed in anticoagulant tubes containing sodium heparin. The samples were immediately centrifuged at 5000 rpm at low temperature (4°C) for 10 min to obtain plasma samples. The samples were stored in a -80°C freezer, awaiting analysis. Three mice were used per time point.
(3) The content of PTE-2 and pterostilbene in the peripheral blood was detected by LC/MS.

### Main Instruments

ExionLC high performance liquid chromatography system, SCIEX;
Triple Quad^{™} 4500 tandem triple quadrupole mass spectrometer, SCIEX;
MS105DU microbalance (1/100,000), Mettler-Toledo;
S1-0256 circular oscillating mixer, IKA;
JXFSTPRP-CL cryogenic tissue grinder, Shanghai Jingxin;
Eppendorf Centrifuge 5425R compact benchtop high-speed refrigerated centrifuge, Eppendorf.

### Detection Conditions

### Liquid chromatography conditions:

| | | | | | |
|---|---|---|---|---|---|
| Autosampler: | ExionLC-AD | | | | |
| Liquid Chromatograph: | ExionLC-AD | | | | |
| Column Oven: | ExionLC-AC | | | | |
| System Control Module: | ExionLC | | | | |
| Chromatographic column: | CORTECS^{®} C18, 2.7 µm (2.1×50 mm) | | | | |
| Column Temperature: | 40°C | | | | |
| Injection volume: | PTE-2: 1 µL | | | | |
| | Pterostilbene: 8 µL | | | | |
| Mobile phase: | A: H₂O (containing 0.1% formic acid) | | | | |
| | B: Acetonitrile (containing 0.1% formic acid) | | | | |

| PTE-2 Gradient: | Time (min) | A (%) | B (%) | Flow (mL/min) | Rate |
|---|---|---|---|---|---|
| | | | | | |
| | 0 | 95 | 5 | 0.6 | |
| | 1.0 | 0 | 100 | 0.6 | |
| | 1.5 | 0 | 100 | 0.6 | |
| | 1.6 | 95 | 5 | 0.6 | |
| | 2.0 | 95 | 5 | 0.6 | |
| | | | | | |

| Pterostilbene gradient: | Time (min) | A (%) | B (%) | Flow (mL/min) | Rate |
|---|---|---|---|---|---|
| | 0 | 90 | 10 | 0.4 | |
| | 0.3 | 90 | 10 | 0.4 | |
| | 1.5 | 0 | 100 | 0.4 | |
| | 2.0 | 0 | 100 | 0.4 | |
| | 2.1 | 90 | 10 | 0.4 | |
| | 3.0 | 90 | 10 | 0.4 | |

### Mass spectrometry conditions:

| | | | | | | |
|---|---|---|---|---|---|---|
| Instrument: | Triple Quad^{™} 4500 (Sciex; Framingham, MA, USA) | | | | | |
| Ion source: | Electrospray ionization source | | | | | |
| Ionization mode: | Electrospray | | | | | |
| Scanning mode: | MRM | | | | | |
| Ion mode: | PTE-2: positive ion mode; Pterostilbene: negative ion mode | | | | | |

| MS/MS information | | | | | | |
|---|---|---|---|---|---|---|
| Analyte | Q1 (m/z) | Q3 (m/z) | DP (v) | EP (v) | CE (v) | CXP (v) |
| PTE-2 | 328.1 | 72.2 | 95 | 10 | 40 | 15 |
| Pterostilbene | 255.0 | 196.8 | -94 | -10 | -41 | -11 |
| Tolbutamide (IS) | 271.3 | 155.0 | 88 | 10 | 17 | 15 |
| Tolbutamide (IS) | 269.1 | 169.7 | -68 | -10 | -23 | -11 |

| | | | | | | |
|---|---|---|---|---|---|---|
| Note: Tolbutamide was used as the internal standard for calculating the peak area ratio. | | | | | | |

The main pharmacokinetic parameters after single oral gavage and intravenous administration of pterostilbene are shown in the table below.

| Parameter | Unit | PTE-2 | | Pterostilbene | |
|---|---|---|---|---|---|
| | | iv (20 mg/kg) | po(100mg/kg) | iv (20 mg/kg) | po(100mg/kg) |
| T_{1/2} | hr | 3.195 | 4.91 | 3.433 | 4.17 |
| Tₘₐₓ | hr | - | 0.50 | - | 0.50 |
| Cₘₐₓ | ng/mL | - | 689 | - | 901 |
| C₀ | ng/mL | 1516 | - | 2294 | - |
| AUC₀₋ₜ | hr*ng/mL | 1134 | 1585 | 4265 | 2357 |
| AUC_{0-∞} | hr*ng/mL | 1135 | 1622 | 4282 | 2384 |
| Vd | mL/kg | 27403 | - | 7806 | - |
| Cl | mL/hr/kg | 5945 | - | 1576 | - |
| MRT₀₋ₜ | hr | 1.15 | 4.91 | 3.23 | 4.16 |
| F | % | - | 27.94 | - | 11.05 |

Thus, the compound of the present disclosure has significantly higher bioavailability.

The implementation of the technical solution of the present disclosure has been exemplarily described above. It should be understood that the protection scope of the present disclosure is not limited to the above-described implementations. Any modification, equivalent replacement, improvement, and the like made by a person skilled in the art without departing from the spirit and principle of the present disclosure shall fall within the protection scope of the claims of the present application.

## Claims

1. A compound of formula (I) or a pharmaceutically acceptable salt thereof:

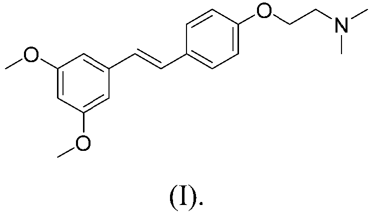

2. The compound or a pharmaceutically acceptable salt thereof according to claim 1, **characterized in that** the pharmaceutically acceptable salt is an acid addition salt formed from the compound of formula (I) and the following inorganic acid or organic acid: the inorganic acid is selected from hydrochloric acid, hydrofluoric acid, hydrobromic acid, hydroiodic acid, sulfuric acid, pyrosulfuric acid, phosphoric acid, or nitric acid, or a hydrogen sulfate; the organic acid is selected from formic acid, acetic acid, acetoacetic acid, pyruvic acid, trifluoroacetic acid, propionic acid, butyric acid, hexanoic acid, heptanoic acid, undecanoic acid, lauric acid, benzoic acid, salicylic acid, 2-(4-hydroxybenzoyl)benzoic acid, camphoric acid, cinnamic acid, cyclopentanepropionic acid, digluconic acid, 3-hydroxy-2-naphthoic acid, nicotinic acid, pamoic acid, pectinic acid, persulfuric acid, 3-phenylpropionic acid, picric acid, pivalic acid, 2-hydroxyethanesulfonic acid, itaconic acid, sulfamic acid, trifluoromethanesulfonic acid, dodecylsulfuric acid, ethanesulfonic acid, benzenesulfonic acid, p-toluenesulfonic acid, methanesulfonic acid, 2-naphthalenesulfonic acid, naphthalenedisulfonic acid, camphorsulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptonic acid, glycerophosphoric acid, aspartic acid, sulfosalicylic acid, hemisulfuric acid, or thiocyanic acid; preferably fumaric acid.

3. The compound or a pharmaceutically acceptable salt thereof according to claim 1 or 2, **characterized in that** the pharmaceutically acceptable salt has the following structure:

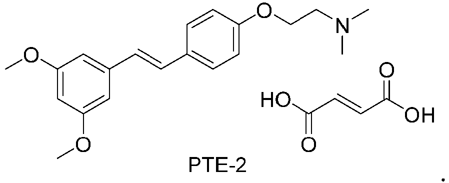

4. A method for preparing the compound according to any one of claims 1-3 or a pharmaceutically acceptable salt thereof, comprising the following step: reacting compound 1 with compound 2 to obtain a compound of formula (I); 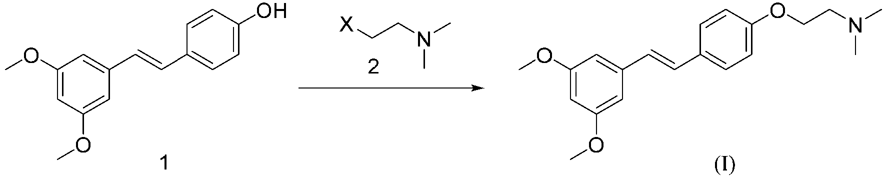 wherein X is halogen, for example Cl, Br, and I.

5. A method for preparing the compound according to any one of claims 1-3 or a pharmaceutically acceptable salt thereof, comprising the following step: mixing a compound of formula (I) with an acid in a solvent to obtain the pharmaceutically acceptable salt; wherein the acid has the definition according to claim 2.

6. The preparation method according to claim 5, **characterized in that** the preparation method comprises: dissolving a compound of formula (I) in a solvent, then adding an acid and continuing the reaction to obtain the pharmaceutically acceptable salt according to claim 1.

7. The preparation method according to claim 5, **characterized in that** the preparation method comprises: dissolving a compound of formula (I) in ethyl acetate, and adding fumaric acid to react to obtain a fumarate salt thereof.

8. A pharmaceutical composition, comprising the compound according to any one of claims 1-3 or a pharmaceutically acceptable salt thereof.

9. Use of the compound according to any one of claims 1-3 or a pharmaceutically acceptable salt thereof, or the pharmaceutical composition according to claim 8, in the preparation of an anti-inflammatory drug.

10. The use according to claim 9, **characterized in that** the use is in the preparation of a drug for treating inflammatory bowel disease.
